# EUROPEAN PATENT APPLICATION

(11) **EP 2 389 928 A2**
(43) Date of publication of application: **30.11.2011**
(21) Application number: 11250555.7
(22) Date of filing: 26.05.2011
(51) Int. Cl.: A61K 9/16, A61K 9/70, A61K 31/445, A61L 27/18

(54) **Biodegradable polymer encapsulated microsphere particulate film and method of making thereof**

(30) Priority: 27.05.2010 US 348846 P; 07.04.2011 US 81531
(71) Applicant: Tyco Healthcare Group LP, New Haven, CT 06511 (US)
(72) Inventor: Griffin, Jeremy, West Hartford, CT 06107 (US); Elachchabi, Amin, Hamden, CT 06518 (US); Stopek, Joshua, Guilford, CT 06437 (US)
(74) Representative: Soames, Candida Jane

(57) **Abstract**

Films containing microsphere particles and processes for forming thereof are disclosed. The microsphere particles encapsulate one or more bioactive agents and may be deposited on a surface of medical devices or be used to form a medical device.

## Description

### CROSS-REFERENCE TO RELATED APPLICATION

This application claims the benefit of and priority to U.S. Provisional Patent Application No. 61/348,846, filed May 27, 2010, the entire disclosure of which is incorporated by reference herein.

### TECHNICAL FIELD

The present disclosure relates to drug delivery vehicles. More particularly, the present disclosure relates to films containing biodegradable polymer microsphere particles that encapsulate one or more bioactive agents.

### BACKGROUND

Surgical implants, such as drug eluting devices, are known to serve as vehicles for the delivery of drugs or other therapeutic agents. Typically, devices, such as stents and meshes, are coated with a biologically active agent to provide treatment to an implant site. It is generally desirable that an effective therapeutic amount of a selected drug be released from the device at a constant rate for an extended period of time. The release of the drug from the coating medium may be dependent upon the nature of the coating material and the drug that is incorporated therein, with drug release occurring by solid state and water diffusion through the coating material or with degradation of the coating material or polymer itself.

### SUMMARY

According to one embodiment of the present disclosure, a medical device is provided. The medical device includes a film having a plurality of poly(lactic-co-glycolic acid) microsphere particles disposed therein, wherein the plurality of poly(lactic-coglycolic acid) microsphere particles encapsulate at least one bioactive agent; and a film former.

According to another embodiment of the present disclosure, a film is provided. The film includes a dried emulsion having a first component including a water-soluble drug and a first polar solvent; a second component including an aliphatic-polyester and a non-polar solvent; and a third component including a water-soluble polymer and a second polar solvent.

A process is also contemplated by the present disclosure. The process includes forming a first, non-polar composition containing at least one biodegradable polymer, at least one bioactive agent and at least one non-polar solvent; forming a second, polar composition containing at least one emulsifier and at least one polar solvent; forming an emulsion by contacting the first, non-polar composition and the second, polar composition; homogenizing the emulsion to form microsphere particles comprising the at least one biodegradable polymer encapsulating the at least one bioactive agent; and drying the homogenized emulsion to form a film including the microsphere particles disposed therein.

A process is also contemplated by the present disclosure. The process includes forming a mixture by contacting a first, non-polar composition comprising at least one biodegradable polymer and at least one non-polar solvent with a second, polar composition comprising at least one bioactive agent in at least one polar solvent; forming an emulsion by contacting the mixture with at least one emulsifier; homogenizing the emulsion to form microsphere particles comprising the at least one biodegradable polymer encapsulating the at least one bioactive agent; and drying the homogenized emulsion to form a film including the microsphere particles disposed therein.

### BRIEF DESCRIPTION OF THE DRAWINGS

Various embodiments of the present disclosure will be described herein below with reference to the figures wherein:

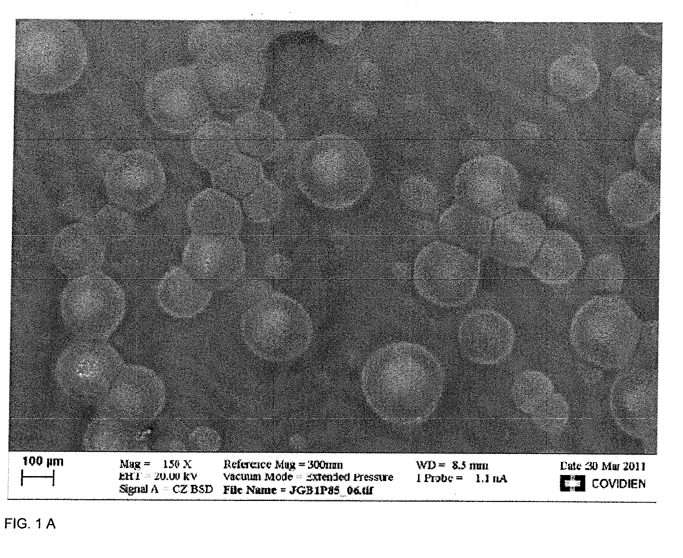

FIG. 1A-1B are Scanning Electron Microscope images of a film in accordance with the present disclosure.

### DETAILED DESCRIPTION

The present disclosure provides for compositions and methods of fabricating films containing microsphere particles formed from a biodegradable polymer that encapsulate one or more bioactive agents. The films may be applied to cover medical devices (e.g., implants, staples, etc.) or may be formed into various types of implants for therapeutic treatment of tissue. In embodiments, the microsphere films may be cast directly from a microsphere mixture directly onto the medical devices in a single process step, thereby avoiding resuspension of microsphere particles to form homogenous suspensions suitable for coating medical devices. The film containing microsphere particles may be formed in a homogenous suspension (e.g., emulsion) of at least one biodegradable polymer, at least one bioactive agent and at least one emulsified container in polar and non-polar compositions.

In embodiments, the microsphere film may be prepared using a single-emulsion (e.g., water-in-oil or oil-in-water emulsion) method, in which a bioactive agent and a biodegradable polymer are non-polar (e.g., water-immiscible or hydrophobic). In embodiments, the bioactive agent may be a water-soluble drug, including, but not limited to bupivacaine hydrochloride (HCI), 5-fluorouracil, doxorubicin, ropivacaine HCl, and combinations thereof. Suitable biodegradable polymers include aliphatic polyesters, such as poly(lactic-co-glycolic acid) ("PLGA") having the following formula:

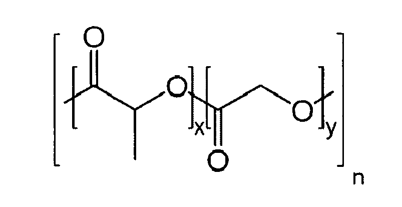

where x may be from about 50 to about 70, in embodiments from about 52 to about 60, y is from about 30 to about 50, in embodiments from about 32 to about 40, and n is from about 20 to about 1000 in embodiments from about 300 to about 500. The molecular weight of the PLGA biodegradable polymer may be from about 30,000 Da to about 120,000 Da, in embodiments from about 32,000 Da to about 60,000 Da.

The biodegradable polymer and the hydrophobic bioactive agent may be dissolved in a non-polar solvent to form a non-polar composition. The bioactive agent may be present in an amount from about 1 % by weight to about 10 % by weight of the non-polar solvent, in embodiments from about 2 % by weight to about 5 % by weight of the non-polar solvent. The biodegradable polymer may be present in an amount from about 1 % by weight to about 10 % by weight of the non-polar solvent, in embodiments from about 2 % by weight to about 5 % by weight of the non-polar solvent, in embodiments about 1 % by weight of the non-polar solvent.

Suitable non-polar solvents include methylene chloride, perchloroethane, trichloroethylene, hexafluoroisopropanol (HFIP), chloroform and combinations thereof. An emulsion is formed by combining the non-polar composition containing the biodegradable polymer and the bioactive agent in the non-polar solvent with a polar composition having an emulsifier, which is also used as a film former, dissolved in a polar solvent. Suitable polar solvents include water (e.g., deionized water), saline and alcohols that are not soluble in non-polar solvents, examples of which include methanol, ethanol and the like, and combinations thereof. It should be noted that both solvents should be immiscible. The emulsifier may be present in an amount from about 1 % by weight to about 10 % of the polar solvent, in embodiments from about 2 % by weight to about 5 % by weight of the polar solvent.

The emulsifier also acts as a microsphere stabilizer agent and as a film former. Suitable emulsifiers include water-soluble polymers, such as, polyvinyl alcohol ("PVA"), polyvinyl pyrrolidone (PVP), polyethylene glycol (PEG), polypropylene glycol (PPG), PLURONICS™, TWEENS™, polysaccharides and combinations thereof. PVA may be used in various hydrolyzed states from about 80% to about 99%, in embodiments, from about 85 % to about 95 %. The melting point of the emulsifier, namely PVA, varies with the hydrolyzation of the compound. Thus, hydrolyzation also affects the solubility of the emulsifier. A substantially fully hydrolyzed PVA (e.g., 99%) may need to be heated to about 75 °C to be soluble in water, whereas 80% hydrolyzed PVA is soluble at room temperature, such as from about 20°C to about 25°C. Blends of various hydrolyzed PVA compounds and other emulsifiers may be used to adjust rate of dissolution of formed films to adjust drying of the resulting microsphere films.

The non-polar composition of the biodegradable polymer and/or the bioactive agent may also include a surfactant to stabilize formed microsphere particles and increase bioactive agent loading efficiency. One, two, or more surfactants may be utilized. Examples surfactants that can be utilized include, for example, polyacrylic acid, methalose, methyl cellulose, ethyl cellulose, propyl cellulose, hydroxy ethyl cellulose, carboxy methyl cellulose, polyoxyethylene cetyl ether, polyoxyethylene lauryl ether, polyoxyethylene octyl ether, polyoxyethylene octylphenyl ether, polyoxyethylene oleyl ether, polyoxyethylene sorbitan monolaurate, polyoxyethylene stearyl ether, polyoxyethylene nonylphenyl ether, dialkylphenoxy poly(ethyleneoxy) ethanol, and polyoxamers.

The microsphere film may also be prepared using a double-emulsion method (e.g., water-in-oil-in-water emulsion, in which the bioactive agent is polar (e.g., hydrophilic). A polar (e.g., aqueous) composition including a solution of the bioactive agent in a first polar solvent is contacted with a non-polar composition including a solution of the biodegradable polymer in a non-polar solvent. The emulsion is then mixed with a polar solution of the emulsifier in a second polar solvent, which may be the same or different as the first polar solvent. In embodiments, the non-polar composition may include a second bioactive agent, which is hydrophobic, allowing for forming emulsions having hydrophobic and hydrophilic bioactive agents.

The bioactive agent may be present in an amount from about 1 % by weight to about 10 % by weight of the first polar solvent, in embodiments, from about 2 % by weight to about 5 % by weight of the first polar solvent. The biodegradable polymer may be present in an amount from about 1 % by weight to about 10 % by weight of the non-polar solvent, in embodiments, from about 2 % by weight to about 5 % by weight of the non-polar solvent. The emulsifier may be present in an amount from about 1 % by weight to about 10 % of the second polar solvent, in embodiments from about 2 % by weight to about 5 % by weight of the second polar solvent.

The loading efficiency of the bioactive agent can also be modulated by adjusting hydrophobic and/or hydrophilic properties of the bioactive agent. In embodiments, the ratio of the salt form to the free base form may be adjusted to obtain a desired loading efficiency. As discussed above, one example of a bioactive agent is bupivacaine HCl. Bupivacaine has a pKa of about 8.1. The salt form of bupivacaine with HCI is illustrated below: 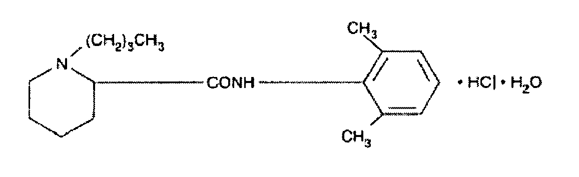 The free base form of bupivacaine, illustrated below, is more hydrophobic than the salt form and thus has a lower aqueous solubility.

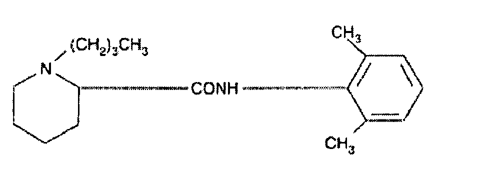

The greater the percentage of the bioactive agent in free base form, i.e., the more the equilibrium is shifted toward the free base form, the slower the overall release rate of the bioactive agent. The salt form of bupivacaine is soluble in water, whereas the free base form is soluble in the water-immiscible solvents used to dissolve the biodegradable polymer.

Once the polar and non-polar compositions are mixed, the mixture is homogenized to form an emulsion. Homogenization may be accomplished by stirring the emulsion at from about 500 revolutions per minute ("rpm") to about 10,000 rpm. In embodiments, the emulsion may be stirred from about 600 rpm to about 9,500 rpm. The emulsion may be stirred for a period of time from about 30 seconds to about 4 hours, in embodiments, from about 90 seconds to about 1 hour. Stirring may also be used to remove the hydrophobic solvent from the mixture, retaining the microsphere particles suspended in an aqueous solution of the emulsifier.

After the solvent is evaporated, the emulsion retains the microsphere particles formed from the biodegradable polymer encapsulating the bioactive agent. The solution also includes free unencapsulated portion of the bioactive agent that is suspended in the emulsion. The microsphere particles may be from about 1 µm to about 200 µm. In embodiments, the microsphere particles may be from about 20 µm to about 60 µm. Size of the microsphere particles may be tailored by modulating the duration and the speed of homogenization (e.g., stirring of the solutions); altering ratio of continuous to discontinuous phase; sheer rate; and the molecular weight and concentrations of polymers and surfactants.

The emulsion may be used to form a medical device by solution-casting the emulsion into molds or spraying the emulsion to form the medical device (e.g., implant), as a result, the emulsion dries out forming a medical device from the film containing the microsphere particles. Cast or sprayed films may be either continuous or discontinuous and may be used in conjunction with other films such as carboxymethyl cellulose, glycerol, multilaminar lactones and combinations thereof. The liquid solvent of the emulsion may then be evaporated by venting, shaking, and other suitable methods to obtain a formed medical device.

In embodiments, the emulsion may be deposited onto a preformed medical device or a portion thereof to form a film having the microsphere particles directly on the surface of the medical device. The film may be incorporated onto the medical device by applying the emulsion to the surface of the device, or portion thereof, utilizing any suitable method known to those skilled in the art. Some examples include, but are not limited to, spraying, dipping, layering, calendaring, etc.

The biodegradable film containing biodegradable polymer microsphere particles that encapsulate one or more bioactive agents also provides for a desired bioactive agent release profile, such as an initial bolus release from the bioactive agent encapsulated within the microsphere particles followed by a sustained release for up to five days from the film which includes the unencapsulated portion of bioactive agent. Where the microsphere particles are formed from a biodegradable polymeric material, the bioactive agent may be released into the body within a period of time from about 1 hour to about 21 days following implantation. In one embodiment, the bioactive agent may be released for about 2 days to about 14 days following implantation.

The medical device may be any surgical implant, such as meshes, scaffolds, grafts, stents, sutures, patches, slings, buttresses, scaffolds, pledgets, and in general, soft tissue repair devices, surgical prostheses and artificial organs; or may be topically applied medical products, such as wound dressings, coverings, tapes, gauzes, and the like, that can be used in medical/surgical procedures. In embodiments, the medical device may include pores or openings over at least a portion of a surface thereof, within which the film may be deposited. The pores may be present as a surface characteristic or a bulk material property, which partially or completely penetrates the medical device, and may be uniformLy distributed across portions thereof. The medical device may have an open-cell structure, where the pores are connected to each other, forming an interconnected network. Conversely, the medical device may have a closed-cell structure, where the pores are not interconnected. Additional pore distribution patterns and configurations are within the purview of those skilled in the art. The pores may be created using any suitable technique, including, but not limited to, lyophilization or freeze-drying, sintering, leaching of salt, and sugar or starch crystals. Alternatively, openings may be formed in filamentous medical devices, such as sutures and meshes, via the spaces formed between the filaments.

The term "bioactive agent", as used herein, is used in its broadest sense and includes any substance or mixture of substances that have clinical use. Consequently, bioactive agents may or may not have pharmacological activity per se, e.g., a dye. A bioactive agent may be any agent that provides a therapeutic or prophylactic effect; a compound that affects or participates in tissue growth, cell growth and/or cell differentiation; a compound that may be able to invoke or prevent a biological action such as an immune response; or a compound that could play any other role in one or more biological processes. Moreover, any agent which may enhance tissue repair, limit the risk of sepsis, modulate the mechanical properties of the medical device, and/or deliver pharmaceutical agents may be applied to the device. A single bioactive agent may be utilized or, in alternate embodiments, a variety of bioactive agents may be incorporated into the medical devices of the present disclosure.

Examples of bioactive agents and alternative forms of these bioactive agents such as salt forms, free acid forms, free base forms, and hydrates include: antimicrobials (e.g., cephalosporins such as cefazolin sodium, cephradine, cefaclor, cephapirin sodium, ceftizoxime sodium, cefoperazone sodium, cefotetan disodium, cefuroxime azotil, cefotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium); penicillins (e.g., ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium); erythromycins (e.g., erythromycin ethylsuccinate, erythromycin, erythromycin estolate, erythromycin lactobionate, erythromycin stearate, and erythromycin ethylsuccinate); and tetracyclines (e.g., tetracycline hydrochloride, doxycycline hyclate, and minocycline hydrochloride, azithromycin, and clarithromycin); analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloride, morphine, oxycodone, codeine, dihydrocodeine bitartrate, pentazocine, hydrocodone bitartrate, levorphanol, diflunisal, trolamine salicylate, nalbuphine hydrochloride, mefenamic acid, butorphanol, choline salicylate, butalbital, phenyltoloxamine citrate, diphenhydramine citrate, methotrimeprazine, cinnamedrine hydrochloride, and meprobamate); anesthetics; antiepileptics; antihistamines; non-steroidal anti-inflammatories (e.g., indomethacin, ketoprofen, flurbiprofen, naproxen, ibuprofen, ramifenazone, and piroxicam); steroidal anti-inflammatories (e.g., cortisone, dexamethasone, fluazacort, celecoxib, rofecoxib, hydrocortisone, prednisolone, and prednisone); cardiovascular drugs (e.g., coronary vasodilators and nitroglycerin); diagnostic agents; cholinomimetics; antimuscarinics; muscle relaxants; adrenergic neuron blockers; neurotransmitters; antineoplastics (e.g., cyclophosphamide, actinomycin, bleomycin, daunorubicin, doxorubicin hydrochloride, epirubicin, mitomycin, methotrexate, fluorouracil, carboplatin, carmustine (BCNU), methyl-CCNU, cisplatin, etoposide, camptothecin and derivatives thereof, phenesterine, paclitaxel and derivatives thereof, docetaxel and derivatives thereof, vinblastine, vincristine, tamoxifen, and piposulfan); immunogenic agents; immunosuppressants (e.g., cyclosporine, azathioprine, mizoribine, and FK506 (tacrolimus)); gastrointestinal drugs; diuretics; lipids; lipopolysaccharides; polysaccharides; enzymes; non-steroidal antifertility agents; parasympathomimetic agents; psychotherapeutic agents; psychoactive drugs; tranquilizers; decongestants; sedative hypnotics (e.g., barbiturates such as pentobarbital and secobarbital); and benzodiazapines such as flurazepam hydrochloride, triazolam, and midazolam); steroids; sulfonamides; vitamins; antimalarials; anti-migraine agents (e.g., ergotamine, propanolol, isometheptene mucate, and dichloralphenazone); anti-parkinson agents (e.g., L-Dopa and ethosuximide); antitussives; bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate); anticholinergic agents (e.g., oxybutynin and ipratropium bromide); xanthines (e.g., aminophylline, dyphylline, metaproterenol sulfate, and aminophylline); mast cell stabilizers (e.g., cromolyn sodium); inhalant corticosteroids (e.g., beclomethasone dipropionate, beclomethasone dipropionate monohydrate, salbutamol, ipratropium bromide, budesonide, ketotifen, salmeterol, xinafoate, terbutaline sulfate, triamcinolone, theophylline, nedocromil sodium, metaproterenol sulfate, flunisolide, and fluticasone proprionate); angiogenic agents; anti-angiogenic agents; alkaloids; analgesics; narcotics (e.g., codeine, dihydrocodeinone, meperidine, morphine, and the like); opoid receptor antagonists (e.g., naltrexone and naloxone); anti-cancer agents; chemotherapeutic drugs; anti-convulsants; anti-emetics (e.g., meclizine hydrochloride, nabilone, prochlorperazine, dimenhydrinate, promethazine hydrochloride, thiethylperazine, and scopolamine); antihistimines (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorpheniramine maleate, and methdilazine); anti-inflammatory agents (e.g., hormonal agents, hydrocortisone, non-hormonal agents, allopurinol, indomethacin, phenylbutzone and the like); prostaglandins and cytotoxic drugs; drugs affecting reproductive organs; estrogens; antibacterials (e.g., amikacin sulfate, aztreonam, chloramphenicol, chloramphenicol palirtate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate); antibodies; antibiotics (e.g., neomycin, streptomycin, chloramphenicol, cephalosporin, ampicillin, penicillin, tetracycline, and ciprofloxacin); anti-fungals (e.g., griseofulvin, ketoconazole, itraconizole, amphotericin B, nystatin, and candicidin); antivirals (e.g., interferon alpha, beta or gamma, zidovudine, amantadine hydrochloride, ribavirin, and acyclovir); anticoagulants (e.g., heparin, heparin sodium, and warfarin sodium); antidepressants (e.g., nefopam, oxypertine, doxepin, amoxapine, trazodone, amitriptyline, maprotiline, phenylzine, desipramine, nortriptyline, tranylcypromine, fluoxetine, doxepin, imipramine, imipramine pamoate, isocarboxazid, trimipramine, and protriptyline); immunological agents; antiasthamatics (e.g., ketotifen and traxanox); antidiabetics (e.g., biguanides and sulfonylurea derivatives); antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine); antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene); antianginal agents such as beta-adrenergic blockers, calcium channel blockers (e.g., nifedipine and diltiazem), and nitrates (e.g., nitroglycerin, isosorbide dinitrate, pentaerythritol tetranitrate, and erythrityl tetranitrate); antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine); antimanic agents (e.g., lithium carbonate); antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, quinidine polygalacturonate, flecainide acetate, tocainide, and lidocaine); antiarthritic agents (e.g., phenylbutazone, sulindac, penicillanine, salsalate, piroxicam, azathioprine, indomethacin, meclofenamate, gold sodium thiomalate, ketoprofen, auranofin, aurothioglucose, and tolmetin sodium); antigout agents (e.g., colchicine and allopurinol); thrombolytic agents (e.g., urokinase, streptokinase, and alteplase); antifibrinolytic agents (e.g., aminocaproic acid); hemorheologic agents (e.g., pentoxifylline); antiplatelet agents (e.g., aspirin); anticonvulsants (e.g., valproic acid, divalproex sodium, phenyloin, phenyloin sodium, clonazepam, primidone, phenobarbitol, carbamazepine, amobarbital sodium, methsuximide, metharbital, mephobarbital, mephenyloin, phensuximide, paramethadione, ethotoin, phenacemide, secobarbitol sodium, clorazepate dipotassium, and trimethadione); agents useful for calcium regulation (e.g., calcitonin and parathyroid hormone); anti-infectives (e.g., GM-CSF); steroidal compounds and hormones (e.g., androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens); progestins (e.g., methoxyprogesterone acetate and norethindrone acetate); corticosteroids (e.g., triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate); thyroid hormones (e.g., levothyroxine sodium); hypoglycemic agents (e.g., human insulin, purified beef insulin, purified pork insulin, glyburide, chlorpropamide, glipizide, tolbutamide, and tolazamide); hypolipidemic agents (e.g., clofibrate, dextrothyroxine sodium, probucol, pravastitin, atorvastatin, lovastatin, and niacin); agents useful for erythropoiesis stimulation (e.g., erythropoietin); and antiulcer/antireflux agents (e.g., famotidine, cimetidine, and ranitidine hydrochloride).

Other examples of suitable bioactive agents, which may be included in the medical device include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids such as antisense molecules, DNA, RNA, and RNAi; oligonucleotides; polynucleotides; and ribozymes.

Other bioactive agents useful in the compositions and methods described herein include mitotane, halonitrosoureas, anthrocyclines, ellipticine, ceftazidime, oxaprozin, valacyclovir, famciclovir, flutamide, enalapril, mefformin, itraconazole, gabapentin, fosinopril, tramadol, acarbose, lorazepan, follitropin, omeprazole, lisinopril, tramsdol, levofloxacin, zafirlukast, granulocyte stimulating factor, nizatidine, bupropion, perindopril, erbumine, adenosine, alendronate, alprostadil, betaxolol, bleomycin sulfate, dexfenfluramine, fentanyl, gemcitabine, glatiramer acetate, granisetron, lamivudine, mangafodipir trisodium, mesalamine, metoprolol fumarate, miglitol, moexipril, monteleukast, octreotide acetate, olopatadine, paricalcitol, somatropin, sumatriptan succinate, tacrine, nabumetone, trovafloxacin, dolasetron, finasteride, isradipine, tolcapone, enoxaparin, fluconazole, lansoprazole, pamidronate, didanosine, diclofenac, cisapride, venlafaxine, troglitazone, fluvastatin, losartan, imiglucerase, donepezil, olanzapine, valsartan, fexofenadine, adapalene, doxazosin mesylate, mometasone furoate, ursodiol, felodipine, nefazodone hydrochloride, valrubicin, albendazole, medroxyprogesterone acetate, nicardipine hydrochloride, zolpidem tartrate, rubitecan, amlodipine besylate/benazepril hydrochloride, paroxetine hydrochloride, podofilox, pramipexole dihydrochloride, quetiapine fumarate, candesartan, cilexetil, ritonavir, busulfan, flumazenil, risperidone, carbemazepine, carbidopa, levodopa, ganciclovir, saquinavir, amprenavir, sertraline hydrochloride, clobustasol, diflucortolone, halobetasolproprionate, sildenafil citrate, chlorthalidone, imiquimod, simvastatin, citalopram, irinotecan hydrochloride, sparfloxacin, efavirenz, tamsulosin hydrochloride, mofafinil, letrozole, terbinafine hydrochloride, rosiglitazone maleate, lomefloxacin hydrochloride, tirofiban hydrochloride, telmisartan, diazapam, loratadine, toremifene citrate, thalidomide, dinoprostone, mefloquine hydrochloride, trandolapril, mitoxantrone hydrochloride, tretinoin, etodolac, nelfinavir mesylate, indinavir, nifedipine, cefuroxime, and nimodipine.

Specific agents within these classes are within the purview of those skilled in the art and are dependent upon such factors as, for example, the type of device in which it is utilized and the tissue being treated. In embodiments, antimicrobial agents such as triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides such as tobramycin and gentamicin; rifampicin; bacitracin; neomycin; chloramphenicol; miconazole; quinolones such as oxolinic acid, norfloxacin, nalidixic acid, pefloxacin, enoxacin and ciprofloxacin; penicillins such as oxacillin and pipracil; nonoxynol 9; fusidic acid; and cephalosporins; may also be used alone or in combination for the treatment of microbial growth. In addition, antimicrobial proteins and peptides, such as lactoferrin and lactoferricin B, and antimicrobial polysaccharides, such as fucans and derivatives thereof, may be included as a bioactive agent in the present disclosure to kill or prevent microbial growth. And anti-adhesive agents may be used to prevent adhesions from forming between the coated medical device and the surrounding tissues. Some examples of these agents include, but are not limited to, poly(vinyl pyrrolidone), carboxymethyl cellulose, hyaluronic acid, alginate, collagen, polyethylene glycol, polyethylene oxide, polypropylene glycol, poly vinyl alcohols, poly acrylic acid, styrene sulfonic acid, polyhydroxyethylmethylacrylate, (pHEMA) and phospholipid vinyls; acrylic polymers such as sodium polyacrylate, polyethylacrylate, and polyacrylamide, polypropylene oxide, phosphorylcholine functional acrylates and methacrylates, homopolymers and combinations thereof.

The term "biodegradable" as used herein is defined to include both bioabsorbable and bioresorbable materials. By biodegradable, it is meant that the material decomposes, or loses structural integrity under body conditions (e.g., enzymatic degradation or hydrolysis) or is broken down (physically or chemically) under physiologic conditions in the body such that the degradation products are excretable or absorbable by the body. Such materials include natural, synthetic, bioabsorbable, and/or non-absorbable materials, as well as combinations thereof.

Representative natural biodegradable polymers which may be used with the bioactive agent include: polysaccharides, such as alginate, dextran, chitin, hyaluronic acid, cellulose, collagen, gelatin, fucans, glycosaminoglycans, and chemical derivatives thereof (substitutions and/or additions of chemical groups, for example, alkyl, alkylene, hydroxylations, oxidations, and other modifications routinely made by those skilled in the art); catgut; silk; linen; cotton; and proteins, such as albumin, casein, zein, silk, and copolymers and blends thereof, alone or in combination with synthetic polymers.

Synthetically modified natural polymers include cellulose derivatives, such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt.

Representative synthetic biodegradable polymers include polyhydroxy acids prepared from lactone monomers, such as glycolide, lactide, caprolactone, ε-caprolactone, valerolactone, and δ-valerolactone, as well as carbonates (e.g., trimethylene carbonate, tetramethylene carbonate, and the like), dioxanones (e.g., 1,4-dioxanone and p-dioxanone), 1,dioxepanones (e.g., 1,4-dioxepan-2-one and 1,5-dioxepan-2-one), and combinations thereof. Polymers formed therefrom include: poly(lactic acid); poly(glycolic acid); poly(trimethylene carbonate); poly(dioxanone); poly(hydroxybutyric acid); poly(hydroxyvaleric acid); poly(lactide-co-(ε-caprolactone)); poly(glycolide-co-(ε-caprolactone)); poly(lactic-co-glycolic acid); polycarbonates; poly(pseudo amino acids); poly(amino acids); poly(hydroxyalkanoate)s; polyalkylene oxalates; polyoxaesters; polyanhydrides; polyortho esters; and copolymers, block copolymers, homopolymers, blends, and combinations thereof.

Other non-limiting examples of biodegradable materials include: aliphatic polyesters; polyethylene glycols; glycerols; copoly (ether-esters); and copolymers, block copolymers, homopolymers, blends, and combinations thereof. Rapidly biodegradable polymers, such as poly(lactide-co-glycolide)s, polyanhydrides, and polyorthoesters, which have carboxylic groups exposed on the external surface as the surface of the polymer erodes, may also be used. It is also envisioned that non-biodegradable polymers may be employed in the present disclosure.

The rate of release of a bioactive agent from the microsphere particles may be controlled by any means within the purview of one skilled in the art. Some examples include, but are not limited to, the thickness of the polymeric components of the microsphere particles and the film; the size of the bioactive agent; the hydrophilicity of the bioactive agent; and the strength of physical and physical-chemical interaction between the bioactive agent, the biodegradable polymer, and/or the medical device material. By properly controlling some of these factors, a controlled release of a bioactive agent from the medical device of the present disclosure can be achieved.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" or "ambient temperature" refers to a temperature of from about 20 ° C to about 25° C.

### EXAMPLE 1 - Preparation of PLGA (70:30) Blue in Water Microsphere particles

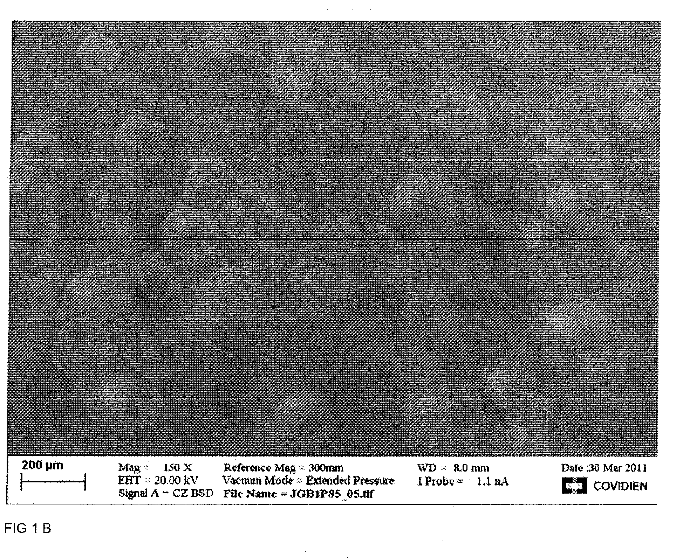

About 8 mL of 1% by weight solution of PLGA (having lactic acid and glycolic acid present in a ratio of about 70:30 and molecular weight (MW) of 120,000 Da available from Covidien of North Haven, CT) in methylene chloride was homogenized for approximately 30 seconds at about 10,000 rotations per minute (rpm). About 2 mL of 1% by weight solution of blue food coloring ("McCormick Blue Food Color" available from McCormick & Company, Inc. of Baltimore, MD) in deionized water was added to the PLGA solution. The resulting solution was homogenized for about 90 seconds at about 10,000 rpm. About 10 mL of 1% by weight solution of PVA in deionized water was added to the PLGA and blue food coloring solution and was homogenized for about 30 seconds at about 10,000 rpm to form an emulsion. The emulsion was then mixed with about 290 mL of 1% by weight aqueous PVA solution. The resulting emulsion was stirred for about 4 hours at about 600 rpm to evaporate methylene chloride. The emulsion was then aliquoted into 50 mL centrifuge tubes and centrifuged at approximately 4,000 rpm for about 5 minutes. As illustrated in FIGS. 1A and 1B, the resulting microsphere particles had a size from about 30 µm to about 200 µm. Size of the microsphere particles was determined with optical microscopy using Zeiss Axiovert 200M fluorescence imaging microscope and Zeiss EVO scanning electron microscope both of which are available from Carl Zeiss Microlmaging GmbH of Munich, DE. Repeating the experiment at higher speed and longer homogenization time (about 12,000-15,000 rpm for about 120-150 seconds) resulted in microsphere particles having a size from about 5 µm to about 30 µm.

### EXAMPLE 2 - Preparation of PLGA (70:30) Bupivacaine in Methanol Microsphere particles

About 8 mL of 1% by weight solution of PLGA (having lactic acid and glycolic acid present in a ratio of about 70:30 and a MW of 120,000 Da available from Covidien of North Haven, CT) in methylene chloride was homogenized for approximately 30 seconds at about 10,000 rpm. About 2 mL of 10% by weight solution of bupivacaine hydrochloride (available from Sigma-Aldrich of St. Louis, MO) in methanol was added to the PLGA solution. The resulting solution was homogenized for about 90 seconds at about 10,000 rpm. About 10 mL of 1% by weight solution of PVA in deionized water was added to the PLGA and bupivacaine solution and was homogenized for about 30 seconds at about 10,000 rpm to form an emulsion. The emulsion was then mixed with about 290 mL of 1% by weight aqueous PVA solution. The resulting emulsion was stirred for about 4 hours at about 600 rpm to evaporate methylene chloride. The emulsion was then aliquoted into 50 mL centrifuge tubes and centrifuged at approximately 4,000 rpm for about 5 minutes. The resulting microsphere particles had a size from about 20 µm to about 60 µm. Repeating the experiment at higher speed and longer homogenization time (about 12,000-15,000 rpm for about 120-150 seconds) resulted in microsphere particles having a size from about 1 µm to about 5 µm.

### EXAMPLE 3 - Preparation of PLGA (50:50) Bupivacaine in Methanol Microsphere particles

About 8 mL of 5% by weight solution of PLGA (having lactic acid and glycolic acid present in a ratio of about 50:50 and a MW of 31,000 - 58,000 Da available from LACTEL Absorbable Polymers of Pelham, AL) in methylene chloride was homogenized for approximately 30 seconds at about 10,000 rpm. About 2 mL of 10% by weight solution of bupivacaine hydrochloride (available from Sigma-Aldrich of St. Louis, MO) in methanol was added to the PLGA solution. The resulting solution was homogenized for about 90 seconds at about 10,000 rpm. About 10 mL of 1% by weight solution of PVA in deionized water was added to the PLGA and bupivacaine solution and was homogenized for about 30 seconds at about 10,000 rpm to form an emulsion. The emulsion was then mixed with about 290 mL of 1% by weight aqueous PVA solution. The resulting emulsion was stirred for about 4 hours at about 600 rpm to evaporate methylene chloride. The emulsion was then aliquoted into 50 mL centrifuge tubes and centrifuged at approximately 4,000 rpm for about 5 minutes. The resulting microsphere particles had a size from about 20 µm to about 60 µm.

### EXAMPLE 4 - Preparation of PLGA (50:50) Bupivacaine in Water Microsphere particles

About 8 mL of 5% by weight solution of PLGA (having lactic acid and glycolic acid present in a ratio of about 50:50 and MW of 31,000 - 58,000 Da available from LACTEL Absorbable Polymers of Pelham, AL) in methylene chloride was homogenized for approximately 30 seconds at about 10,000 rpm. About 1 mL of 10% by weight solution of bupivacaine hydrochloride (available from Sigma-Aldrich of St. Louis, MO) in methanol was added to the PLGA solution. The resulting solution was homogenized for about 90 seconds at about 10,000 rpm. About 10 mL of 1% by weight solution of PVA in deionized water was added to the PLGA and bupivacaine solution and was homogenized for about 30 seconds at about 10,000 rpm to form an emulsion. The emulsion was then mixed with about 290 mL of 1% by weight aqueous PVA solution. The resulting emulsion was stirred for about 4 hours at about 600 rpm to evaporate methylene chloride. The emulsion was then aliquoted into 50 mL centrifuge tubes and centrifuged at approximately 4,000 rpm for about 5 minutes. The resulting microsphere particles had a size from about 20 µm to about 60 µm.

It will be appreciated that of the above-disclosed and other features and functions, or alternatives thereof, may be desirably combined into many other different systems or applications. Also that various presently unforeseen or unanticipated alternatives, modifications, variations or improvements therein may be subsequently made by those skilled in the art which are also intended to be encompassed by the following claims. Unless specifically recited in a claim, steps or components of claims should not be implied or imported from the specification or any other claims as to any particular order, number, position, size, shape, angle, or material.
The invention may be described by reference to the following numbered paragraphs:
1. A medical device comprising a film including a plurality of poly(lactic-co-glycolic acid) microsphere particles disposed therein, wherein the plurality of poly(lactic-co-glycolic acid) microsphere particles encapsulate at least one bioactive agent; and a film former.
2. The medical device according to paragraph 1, wherein the poly(lactic-co-glycolic acid) is of formula: 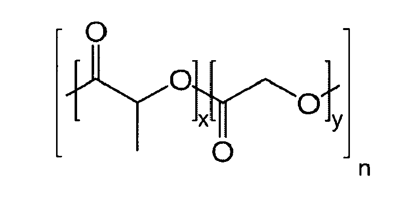 wherein x is from about 50 to about 70, y is from about 30 to about 50, and n is from about 20 to about 1000.
3. The medical device according to paragraph 2, wherein x is from about 50 to about 60, y is from about 32 to about 40, and n is from about 300 to about 500.
4. The medical device according to paragraph 1, wherein the film is formed from a single emulsion.
5. The medical device according to paragraph 1, wherein the film is formed from a double emulsion.
6. The medical device according to paragraph 1, wherein the at least one bioactive agent includes a water-soluble drug.
7. The medical device according to paragraph 6, wherein the water-soluble drug is bupivacaine hydrochloride.
8. The medical device according to paragraph 6, wherein the film former is polyvinyl alcohol hydrolyzed from about 80% to about 99%.
9. A film comprising a dried emulsion including a first component including a water-soluble drug and a first polar solvent, a second component including an aliphatic-polyester and a non-polar solvent; and a third component including a water-soluble polymer and a second polar solvent.
10. The film according to paragraph 9, wherein the aliphatic-polyester is present in an amount from about 1 % by weight to about 5 % by weight of the non-polar solvent.
11. The film according to paragraph 9, wherein the water-soluble drug is present in an amount from about 1 % by weight to about 5 % by weight of the first polar solvent.
12. The film according to paragraph 9, wherein the water-soluble polymer is present in an amount of about 1 % by weight of the second polar solvent.
13. The film according to paragraph 9, wherein the non-polar solvent is methylene chloride.
14. The film according to paragraph 9, wherein the first polar solvent is methanol.
15. The film according to claim 9, wherein the second polar solvent is water.
16. A process comprising forming a first, non-polar composition containing at least one biodegradable polymer, at least one bioactive agent and at least one non-polar solvent, forming a second, polar composition containing at least one emulsifier and at least one polar solvent, forming an emulsion by contacting the first, non-polar composition and the second, polar composition, homogenizing the emulsion to form microsphere particles comprising the at least one biodegradable polymer encapsulating the at least one bioactive agent; and drying the homogenized emulsion to form a film including the microsphere particles disposed therein.
17. The process according to paragraph 16, further comprising removing the at least one non-polar solvent from the emulsion.
18. The process according to paragraph 16, wherein the at least one biodegradable polymer is of formula: 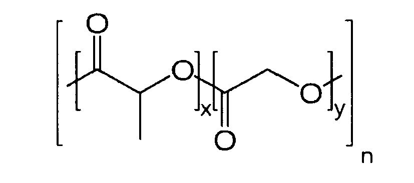 wherein x is from about 50 to about 70, y is from about 30 to about 50, and n is from about 20 to about 1000.
19. The process according to paragraph 18, wherein x is from about 50 to about 60, y is from about 32 to about 40, and n is from about 300 to about 500.
20. The process according to paragraph 16, wherein the at least one emulsifier is polyvinyl alcohol hydrolyzed from about 80% to about 99%.
21. The process according to paragraph 16, further comprising depositing the homogenized emulsion on at least a portion of a medical device prior to drying the homogenized emulsion.
22. A process comprising forming a mixture by contacting a first, non-polar composition comprising at least one biodegradable polymer and at least one non-polar solvent with a second, polar composition comprising least one bioactive agent in at least one polar solvent, forming an emulsion by contacting the mixture with at least one emulsifier, homogenizing the emulsion to form microsphere particles comprising the at least one biodegradable polymer encapsulating the at least one bioactive agent; and drying the homogenized emulsion to form a film including the microsphere particles disposed therein.
23. The process according to paragraph 22, further comprising removing the at least one non-polar solvent from the emulsion.
24. The process according to paragraph 22, wherein the at least one biodegradable polymer is of formula: 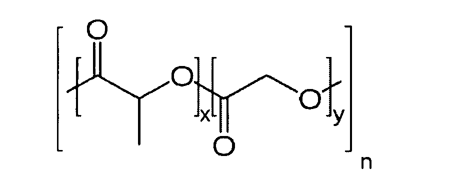 wherein x is from about 50 to about 70, y is from about 30 to about 50, and n is from about 20 to about 1000.
25. The process according to paragraph 24, wherein the at least one emulsifier is polyvinyl alcohol hydrolyzed from about 80% to about 99%.
26. The process according to paragraph 22, further comprising depositing the homogenized emulsion on at least a portion of a medical device prior to drying the homogenized emulsion.

## Claims

1. A medical device comprising:
a film including a plurality of poly(lactic-co-glycolic acid) microsphere particles disposed therein, wherein the plurality of poly(lactic-co-glycolic acid) microsphere particles encapsulate at least one bioactive agent; and
a film former.

2. The medical device according to claim 1, wherein the poly(lactic-co-glycolic acid) is of formula: 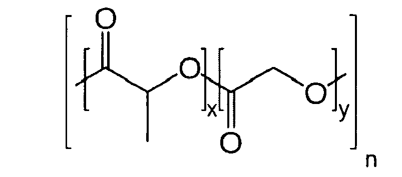 wherein x is from about 50 to about 70, y is from about 30 to about 50, and n is from about 20 to about 1000.

3. The medical device according to claim 2, wherein x is from about 50 to about 60, y is from about 32 to about 40, and n is from about 300 to about 500.

4. The medical device according to claim 1,2 or 3, wherein the film is formed from a single emulsion, or wherein the film is formed from a double emulsion; and/or wherein the at least one bioactive agent includes a water-soluble drug

5. The medical device according to claim 4, wherein the water-soluble drug is bupivacaine hydrochloride; and/or wherein the film former is polyvinyl alcohol hydrolyzed from about 80% to about 99%.

6. A film comprising:
a dried emulsion including:
a first component including a water-soluble drug and a first polar solvent;
a second component including an aliphatic-polyester and a non-polar solvent;
and
a third component including a water-soluble polymer and a second polar solvent.

7. The film according to claim 6, wherein the aliphatic-polyester is present in an amount from about 1 % by weight to about 5 % by weight of the non-polar solvent; and/or wherein the water-soluble drug is present in an amount from about 1 % by weight to about 5 % by weight of the first polar solvent; and/or wherein the water-soluble polymer is present in an amount of about 1 % by weight of the second polar solvent.

8. The film according to claim 7, wherein the non-polar solvent is methylene chloride; and/or wherein the first polar solvent is methanol; and/or wherein the second polar solvent is water.

9. A process comprising:
forming a first, non-polar composition containing at least one biodegradable polymer, at least one bioactive agent and at least one non-polar solvent;
forming a second, polar composition containing at least one emulsifier and at least one polar solvent;
forming an emulsion by contacting the first, non-polar composition and the second, polar composition;
homogenizing the emulsion to form microsphere particles comprising the at least one biodegradable polymer encapsulating the at least one bioactive agent; and
drying the homogenized emulsion to form a film including the microsphere particles disposed therein.

10. The process according to claim 9, further comprising:
removing the at least one non-polar solvent from the emulsion.

11. The process according to claim 9 or claim 10, wherein the at least one biodegradable polymer is of formula: 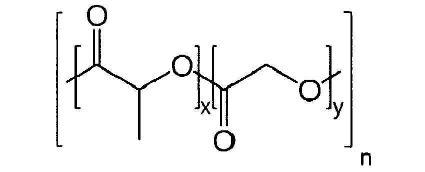 wherein x is from about 50 to about 70, y is from about 30 to about 50, and n is from about 20 to about 1000.

12. The process according to claim 11, wherein x is from about 50 to about 60, y is from about 32 to about 40, and n is from about 300 to about 500.

13. The process according to claim 12, wherein the at least one emulsifier is polyvinyl alcohol hydrolyzed from about 80% to about 99%.

14. The process according to any of claims 9 to 13, further comprising:
depositing the homogenized emulsion on at least a portion of a medical device prior to drying the homogenized emulsion.

15. A process comprising:
forming a mixture by contacting a first, non-polar composition comprising at least one biodegradable polymer and at least one non-polar solvent with a second, polar composition comprising least one bioactive agent in at least one polar solvent;
forming an emulsion by contacting the mixture with at least one emulsifier;
homogenizing the emulsion to form microsphere particles comprising the at least one biodegradable polymer encapsulating the at least one bioactive agent; and
drying the homogenized emulsion to form a film including the microsphere particles disposed therein.

16. The process according to claim 15, further comprising:
removing the at least one non-polar solvent from the emulsion.

17. The process according to claim 16, wherein the at least one biodegradable polymer is of formula: 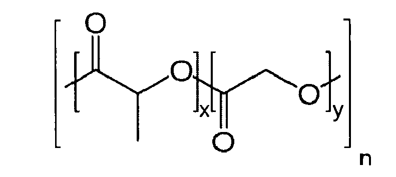 wherein x is from about 50 to about 70, y is from about 30 to about 50, and n is from about 20 to about 1000.

18. The process according to claim 17, wherein the at least one emulsifier is polyvinyl alcohol hydrolyzed from about 80% to about 99%.

19. The process according to claim 18, further comprising:
depositing the homogenized emulsion on at least a portion of a medical device prior to drying the homogenized emulsion.
